(19) Europäisches Patentamt
European Patent Office
Office européen des brevets

(11) Publication number: **0 414 952 A1**

(12) # EUROPEAN PATENT APPLICATION

(21) Application number: **89116152.3**

(22) Date of filing: **31.08.89**

(51) Int. Cl.5: **C07D 339/06**, C07D 409/04, C07D 409/08, C07D 409/12, A61K 31/385

(43) Date of publication of application: **06.03.91 Bulletin 91/10**

(84) Designated Contracting States: **AT BE CH DE ES FR GB GR IT LI LU NL SE**

(71) Applicant: **NIHON NOHYAKU CO., LTD.**
**1-2-5, Nihonbashi**
**Chuo-ku Tokyo(JP)**

(72) Inventor: **Hiraga, Kunikazu**
**4-2-305, Shiginonishi-3-chome**
**Joto-ku Osaka(JP)**
Inventor: **Goto, Makoto**
**Meitsu Kitanoda 409**
**77-1, Nakachaya Sakai-shi(JP)**
Inventor: **Inoue, Kazuyoshi**
**3-16, Akutagawacho-2-chome**
**Takatsuki-shi(JP)**
Inventor: **Uchida, Matazaemon**
**14-22, Daishicho**
**Kawachinagano-shi(JP)**

(74) Representative: **Patentanwälte Grünecker, Kinkeldey, Stockmair & Partner**
**Maximilianstrasse 58**
**W-8000 München 22(DE)**

(54) **Dithiolane derivatives.**

(57) Novel dithiolane derivatives are produced by reacting glyoxal or glyoxal sodium bisulfite adduct with the amine compounds in the presence of the acids and then with dithiolate. The dithiolane derivatives can activate liver functions and are effective for treatment and prophylaxis of hepatitis and other hepatic disorders.

EP 0 414 952 A1

# DITHIOLANE DERIVATIVES

## BACKGROUND OF THE INVENTION

## FIELD OF THE INVENTION

The present invention relates to novel dithiolane derivatives which are effective for treatment and/or prophylaxis of liver or hepatic diseases. The present invention further relates to processes for production thereof and therapeutic compositions for hepatic diseases containing the derivatives as the effective ingredient.

## DISCUSSION ON RELATED ART

It has been disclosed that cyclohexanedione derivatives and dithiolane derivatives shown below are effective for the treatment of liver damage.

1. 2-(1,3-dithiol-2-ylidene)-1,3-cyclohexanedione derivatives are described in U.S. Patent No. 4,668,799 and
2. cyclohexanedione derivative represented by formula:

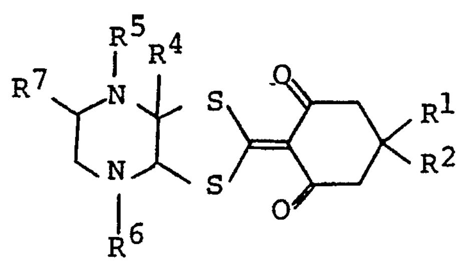

are described in Japanese Patent Application KOKAI (the term "KOKAI" is used herein to refer to an unexamined application which was laid open to public inspection) No. 63-183586;

3. 4,5-substituted dithiolane derivative represented by formula:

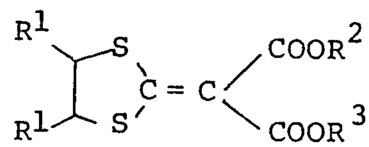

are described in Japanese Patent Application KOKAI No. 63-60983; it is mentioned therein that these compounds are useful for the treatment of liver damage. There is still a desire, however, for a compound capable of curing and/or preventing liver disorders at a considerably lower dosage which will provide a more safety margin for treating both men and animals.

## SUMMARY OF THE INVENTION

An object of the present invention is to provide novel dithiolane derivatives and pharmaceutically acceptable salts thereof, which possess liver function activating activities.

Another object of the present invention is to provide compositions for treating and/or preventing hepatic diseases which comprise as the effective ingredient the dithiolane derivatives described above.

A further object of the present invention is to provide processes for production of these dithiolane derivatives.

A dithiolane derivatives represented by the general formula (I)

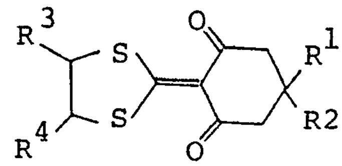

(I)

wherein $R^1$ represent hydrogen atom; $C_1$-$C_6$ alkyl group; phenyl group substituted with 1 to 2 groups selected from the group consisting of halogen atom, $C_1$-$C_4$ alkyl group, $C_1$-$C_4$ alkoxy group, $C_1$-$C_4$ haloalkoxy group, $C_1$-$C_4$ alkylthio group and methylenedioxy group; furyl group which may or not may be substituted with $C_1$-$C_4$ alkyl group, or thienyl group; $R^2$ represent hydrogen atom or $C_1$-$C_4$ alkyl group, $R^3$ represents di($C_1$-$C_4$ alkyl)amino group, N-hydroxyethyl N-methylamino group, formula:

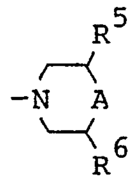

(wherein $R^5$ and $R^6$, which may be the same or different, represent hydrogen atom or $C_1$-$C_4$ alkyl group, A represents nitrogen atom substituted with $C_1$-$C_4$ alkyl group or $C_2$-$C_5$ alkoxycarbonyl group, oxygen atom, methylene group or single bond, $R^4$ have a same meaning as $R^3$, hydroxy group or formula:

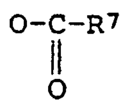

(wherein $R^7$ represents $C_1$-$C_4$ alkyl group, $C_1$-$C_4$ alkoxy group, furyl group, phenyl group, p-chlorophenyl group).

The dithiolane derivatives represented by the general formula (I) are novel compounds that are not found in publications. The dithiolane derivatives exerts, for example, an activating action on liver functions and are thus effective as liver function activators or therapeutic agents for hepatic diseases in human or animals.

## DETAILED DESCRIPTION OF PREFERRED EMBODIMENTS

Preferred substituents for the dithiolane derivatives represented by the general formula (I) in the present invention include, for $R^1$, hydrogen atom; $C_1$-$C_6$ alkyl group; phenyl group; phenyl group substituted with 1 to 2 groups selected from the group consisting of halogen atom, $C_1$-$C_4$ alkyl group and $C_1$-$C_4$ alkoxy group; and furyl group which may or not may be substituted with C-$C_4$ alkyl group; with particularly preferred being hydrogen atom and methyl group. For $R^2$, preferred examples include a hydrogen atom and $C_1$-$C_4$ alkyl group, with particularly preferred being a hydrogen atom and methyl group. For $R^3$, preferred being di-($C_1$-$C_4$ alkyl)amino group, morpholino group or 4-$C_1$-$C_4$ alkylpiperazino group, with particularly preferred being dimethylamino group and morpholino group, $R^4$ represents di($C_1$-$C_4$ alkyl)amino group, morpholino group, hydroxy group, $C_2$-$C_5$ alkylcarbonyl group or $C_2$-$C_5$ alkoxycarbonyl group, with particularly preferred being, dimethylamino group, morpholino group, acetoxy group, propionyloxy group and ethoxycarbonyloxy group.

The compound of general formula (I) can be produced, for example, by methods A, B, C and D as shown in the following scheme:

## Method A

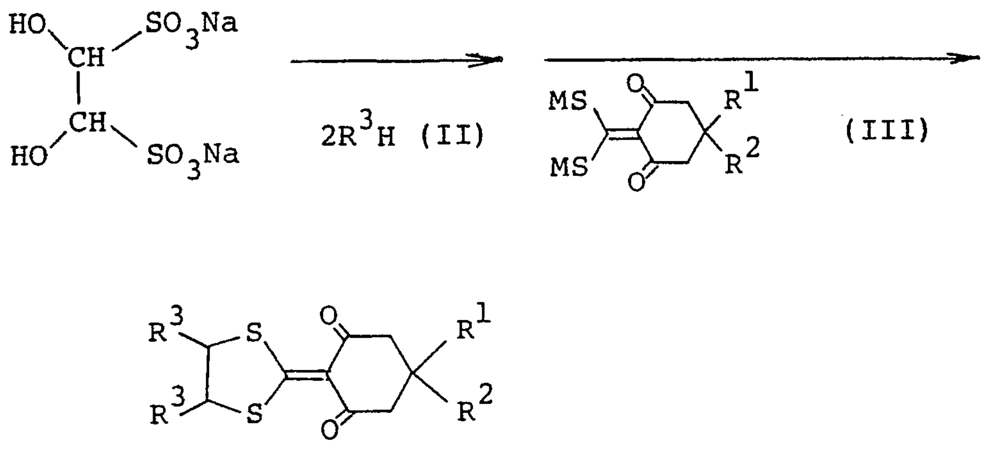


(Ia)

wherein $R^1$, $R^2$ and $R^3$ have the same meaning as defied above, M represents alkali metal atom.

That is, the compound of general formula (I) can be produced by reacting the glyoxal sodium bisulfite adduct represented by the structural formula above with the compound of the general formula (II) in water or a solvent mixture of water and an organic solvent, at a temperature chosen from, e.g., -20° C to 50° C, and then reacting the resulting reaction mixture with the compound represented by the general formula (III) at a temperature chosen from, e.g., -20° C to 80° C.

The glyoxal sodium bisulfite adduct, as shown below, can be obtained by reacting glyoxal with an equivalent or a slightly excess of sodium bisulfite in water at a temperature chosen from, e.g., under cooling to 80° C.

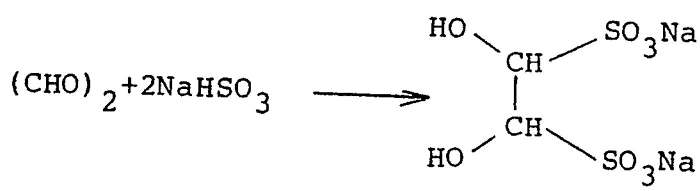


The compound of the general formula (III), as shown below, can be obtained by reacting a compound of general formula (IV) with carbon disulfide in a polar solvent in the presence of a base at a temperature chosen from, e.g., -20° C to 60° C.

In most case, the compound of the general formula (III) can be used without being separated from the reaction mixture.

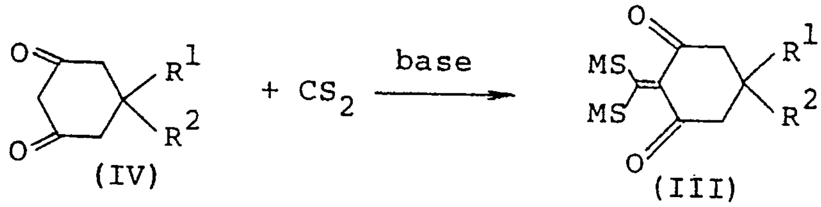


(IV) (III)

wherein $R^1$, $R^2$ and M have the same meaning as defined above.

For a base usable in preparing the compound of the general formula (III), there can be exemplified, for

example, a hydroxide such as sodium hydroxide, potassium hydroxide and a carbonate such as sodium carbonate, potassium carbonate.

For a solvent, there can be exemplified, for example, dimethylformamide, dimethylsulfoxide, hexamethylphosphoramide and N,N-dimethylethyleneurea etc., and in combination of these solvents or in combination of water with above organic solvents.

## Method B

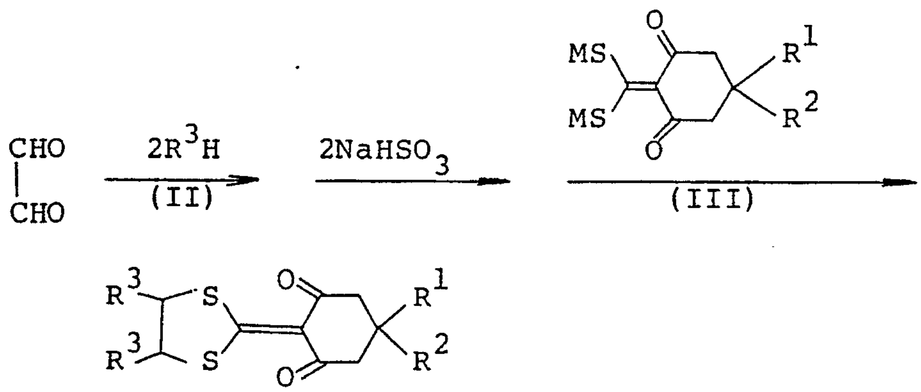


(Ia)

wherein $R^1$, $R^2$, $R^3$ and M have the same meaning as defined above.

That is, the compound of the general formula (Ia) can be produced by reacting glyoxal with the compound of the general formula (II) in a water or in combination of water and organic solvent at a temperature chosen from, e.g., under cooling to room temperature, and then reacting the resulting mixture with sodium bisulfite at a temperature chosen from, e.g., room temperature to 60 ° C and further reacting the resulting mixture with the compound of general formula (III) at a temperature chosen from, e.g., under cooling to 80 ° C.

For a solvent which can be used in Method A or B, there can be exemplified, for example, a polar solvent such as, dimethylformamide, dimethylsulfoxide, hexamethylphosphoramide and N,N-dimethylethyleneurea etc., and in combination of these solvents.

The reaction time may vary depending upon reaction temperature and reaction scale, but can be chosen from 1 to 24 hours.

Each reaction proceeds stoichiometrically so that reactants may be used in eqimolar amounts. However, any of the reactants may also be used in an excess amount, of course.

Process C

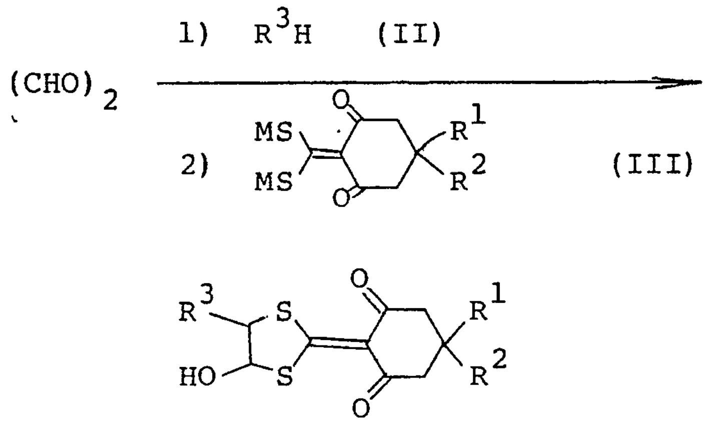


(Ib)

wherein $R^1$, $R^2$, $R^3$ and M are the same meaning as described above.

That is, the compound represented by the general formula (I) can be produced by reacting glyoxal with the compound represented by the general formula (II), in the presence of an acid, in a water or in a combination of water and an organic solvent, at a temperature chosen from under cooling to about room temperature, and then reacting with the compound represented by the general formula (III) at a temperature chosen from, e.g., -20° C to 50° C.

For a solvent which can be used to mixed with water in this process, there can be exemplified, a polar solvent such as, dimethylformamide, dimethylsulfoxide, hexamethylphosphoramide and N,N-dimethylethyleneurea etc., and a mixture solvent of these polar solvents. For the acids used are inorganic acids such as hydrochloric acid, sulfuric acid, sodium bisulfite, etc.; organic acids such as acetic acid, benzenesulfonic acid, toluensulfonic acid, etc.

The reaction time may vary depending upon reaction temperature and reaction scale. In the reaction between glyoxal and the compound represented by the general formula (II), the reaction time is appropriately chosen from the range of 10 minutes to 6 hours. Further at the step where the reaction product is reacted with the compound represented by general formula (III), the reaction time is chosen from the range of 5 minutes to 20 hours.

Each reaction proceeds stoichiometrically so that reactants may be used in equimolar amounts. However, any of the reactants may also be used in an excess amount, of course.

Process D

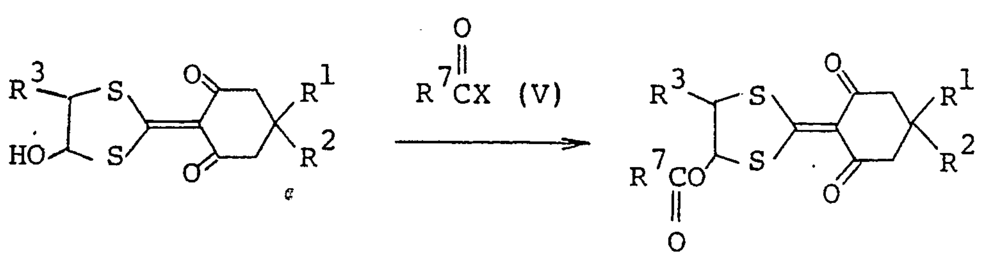


(Ib) (Ic)

wherein $R^1$, $R^2$, $R^3$ and R are the same meaning as described above, and X represents a halogen atom.

That is, the compound represented by the general formula (Ic) can be produced by reacting glyoxal with the compound represented by the general formula (Ib) in an inert solvent at a temperature chosen from, e.g., -20 ° C to 50 ° C, in the presence of a base.

Any solvent can be used in this reaction so long as it dose not interfere with the reaction. There can be exemplified, for example, halogenated hydrocarbon such as methylene dichloride, chloroform, etc.; ethers such as diethyl ether, tetrahydrofuran, dioxane, etc.; aromatic hydrocarbons such as benzene, toluene, xylene, etc.

For a base are organic bases such as triethylamine, tri-n-butylamine, pyridine, DBU (1,8-diazabicyclo (5,4,0)undec-7-ene), etc.

The reaction time may vary depending upon reaction temperature and reactant scale but is appropriately chosen from the range of 5 minutes to 6 hours.

This reaction proceeds stoichiometrically so that the reactants and the base may be used in equimolar amounts. However, any of them may also be used in an excess amount, of course.

Further, the salt of the compound of the general formula (I) was obtained by reacting the compound of the general formula (I) with the acid.

The salt of the compound of the general formula (I) may be any of pharmaceutically acceptable salt. For the acids used to prepare the salt, there exemplified, for example, inorganic acids such as hydrogen chloride, sulfuric acid, phosphoric acid etc., organic carboxylic acids such as acetic acid, succinic acid, fumaric acid, tartaric acid and organic sulfonic acids such as methanesulfonic acid, heptanesulfonic acid, benzenesulfonic acid, toluenesulfonic acid.

For the solvent, there are exemplified, water, alcohol, tetrahydrofuran, acetone, ether, ethyl acetate and the like.

The compound of thegeneral formula (I) and its salt can be separated by the conventional method.

Representative examples of the compounds of the general formula (I) and their salts will be shown in Table 1, but the derivatives are not limited to these examples.

Table 1

| Compound No. | $R^1$ | $R^2$ | $R^3$ | $R^4$ | Physical property (melting point °C) |
|---|---|---|---|---|---|
| 1 | H | H | $-N(CH_3)_2$ | $-N(CH_3)_2$ | m.p. 175 ~ 178°C |
| 2 | H | H | 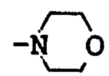 | 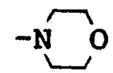 | m.p. 223 ~ 229°C |
| 3 | $CH_3$ | $CH_3$ | $-N(CH_3)_2$ | $-N(CH_3)_2$ | m.p. 178 ~ 179°C |
| 4 | 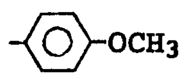 | H | 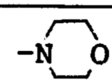 | 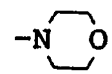 | m.p. 187 ~ 190°C |
| 5 | H | H | $-N(CH_3)_2$ | OH | m.p. 153 ~ 156°C |
| 6 | H | H | $-N(CH_3)_2$ | 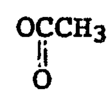 | m.p. 148 ~ 149°C |
| 7 | H | H | $-N(CH_3)_2$ | 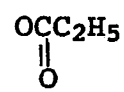 | m.p. 90 ~ 91°C |
| 8 | H | H | $-N(CH_3)_2$ | 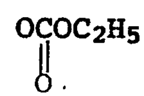 | m.p. 107 ~ 110°C |
| 9 | H | H | $-N(CH_3)_2$ | 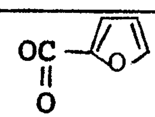 | m.p. 151 ~ 153°C |

- Cont'd -

Table 1 (Cont'd)

| | | | | | |
|---|---|---|---|---|---|
| 10 | H | H | 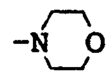 | OH | m.p. 180 ~ 185°C |
| 11 | H | H | 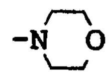 | $OCCH_3$<br>‖<br>O | m.p. 226 ~ 228°C |
| 12 | H | H | 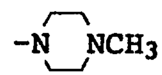 | $OCCH_3$<br>‖<br>O | m.p. 187 ~ 190°C |
| 13 | $CH_3$ | $CH_3$ | $-N(CH_3)_2$ | OH | m.p. 152 ~ 153°C |
| 14 | $CH_3$ | $CH_3$ | 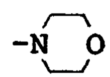 | OH | m.p. 164 ~ 165°C |
| 15 | $CH_3$ | $CH_3$ | 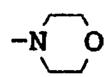 | 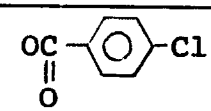 | m.p. 67 ~ 69°C |
| 16 | $CH_3$ | $CH_3$ | $-N(C_2H_5)_2$ | $OCCH_3$<br>‖<br>O | paste |
| 17 | $CH_3$ | $CH_3$ | 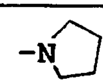 | $OCCH_3$<br>‖<br>O | m.p. 140 ~ 143°C |

- Cont'd -

Table 1 (Cont'd)

| | | | | | |
|---|---|---|---|---|---|
| 18 | $CH_3$ | $CH_3$ | 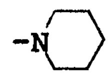 | 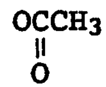 | m.p. 164 ~ 166°C |
| 19 | $CH_3$ | $CH_3$ | 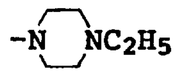 | 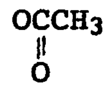 | amorphous |
| 20 | $CH_3$ | $CH_3$ | 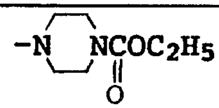 | 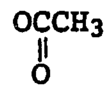 | amorphous |
| 21 | $CH_3$ | $CH_3$ | 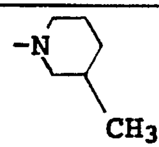 | 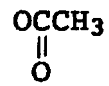 | amorphous |
| 22 | $CH_3$ | $CH_3$ | 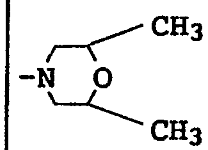 | 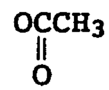 | amorphous |
| 23 | $CH_3$ | $CH_3$ | 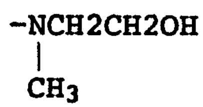 | 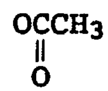 | amorphous |

- Cont'd -

Table 1 (Cont'd)

| | | | | | |
|---|---|---|---|---|---|
| 24 | $C_2H_5$ | H | $-N(CH_3)_2$ | $O\underset{\overset{\|}{O}}{C}CH_3$ | paste |
| 25 | $C_4H_9$-t | $CH_3$ | $-N(CH_3)_2$ | $O\underset{\overset{\|}{O}}{C}CH_3$ | amorphous |
| 26 | 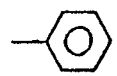 | H | $-N(CH_3)_2$ | OH | m.p. 157 ~ 158°C |
| 27 | 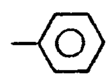 | H | $-N(CH_3)_2$ | $O\underset{\overset{\|}{O}}{C}CH_3$ | m.p. 137 ~ 138°C |
| 28 | 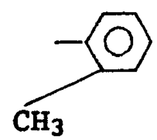 | H | $-N(CH_3)_2$ | OH | m.p. 169 ~ 171°C |
| 29 | 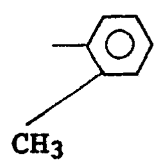 | H | $-N(CH_3)_2$ | $O\underset{\overset{\|}{O}}{C}CH_3$ | m.p. 141 ~ 149°C |

- Cont'd -

Table 1 (Cont'd)

| | | | | | |
|---|---|---|---|---|---|
| 30 | 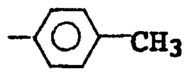 | H | $-N(CH_3)_2$ | 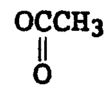 | m.p. 166 ~ 169°C |
| 31 | 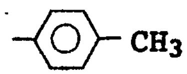 | H | $-N(CH_3)_2$ | 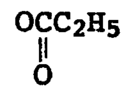 | m.p. 130 ~ 133°C |
| 32 | 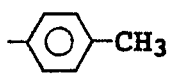 | H | 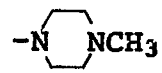 | 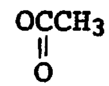 | m.p. 189 ~ 190°C |
| 33 | 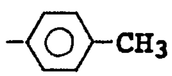 | H | 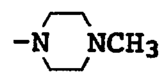 | 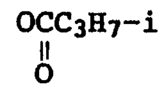 | m.p. 219 ~ 225°C |
| 34 | 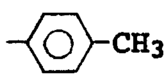 | H | -N NCH3 | 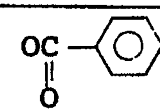 | m.p. 157 ~ 161°C |
| 35 | 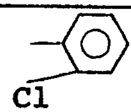 | H | $-N(CH_3)_2$ | 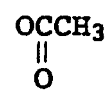 | m.p. 154 ~ 157°C |
| 36 | 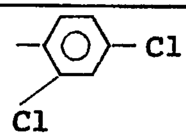 | H | $-N(CH_3)_2$ | 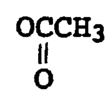 | m.p. 207 ~ 213°C |

- Cont'd -

Table 1 (Cont'd)

| | | | | | |
|---|---|---|---|---|---|
| 37 | 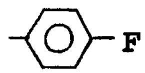 | H | $-N(CH_3)_2$ | 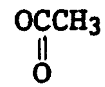 | m.p. 89 ~ 93°C |
| 38 | 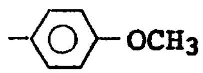 | H | $-N(CH_3)_2$ | OH | m.p. 147 ~ 149°C |
| 39 | 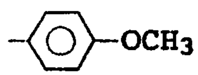 | H | $-N(CH_3)_2$ | 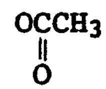 | m.p. 186 ~ 189°C |
| 40 | 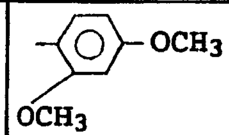 | H | $-N(CH_3)_2$ | 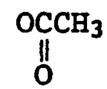 | m.p. 120 ~ 122°C |
| 41 | 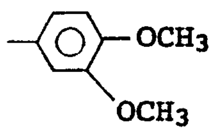 | H | $-N(CH_3)_2$ | 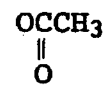 | paste |
| 42 | 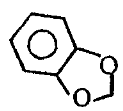 | H | $-N(CH_3)_2$ | 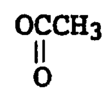 | m.p. 217 ~ 220°C |

- Cont'd -

Table 1 (Cont'd)

| | | | | | |
|---|---|---|---|---|---|
| 43 | 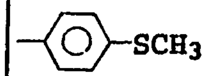 | H | 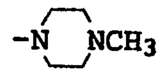 | 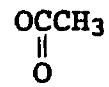 | m.p. 105 ~ 112°C |
| 44 | 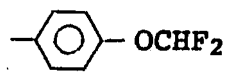 | H | $-N(CH_3)_2$ | 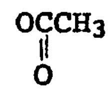 | m.p. 136 ~ 139°C |
| 45 | 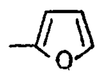 | H | $-N(CH_3)_2$ | 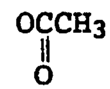 | m.p. 159 ~ 160°C |
| 46 | 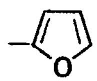 | H | 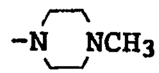 | 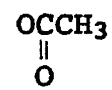 | amorphous |
| 47 | 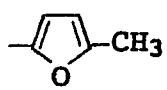 | H | $-N(CH_3)_2$ | OH | m.p. 125 ~ 126°C |
| 48 | 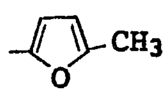 | H | $-N(CH_3)_2$ | 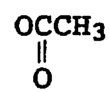 | paste |

- Cont'd -

Table 1 (Cont'd)

| | | | | | |
|---|---|---|---|---|---|
| 49 | 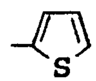 | H | $-N(CH_3)_2$ | $OCCH_3$ ‖ O | m.p. 155 ~ 156.5°C |
| 50 | 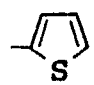 | H | $-N(CH_3)_2$ | $OCCH_3$ ‖ O | m.p. 180 ~ 181°C |
| 51 | 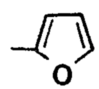 | H | $-N(CH_3)_2$ | OH | m.p. 121 ~ 132°C |

Next, in Table 2 are shown NMR spectra data of the compounds whose physical properties are expressed in term "paste or amorphous" in Table 2.

Table 2

| Compound No. | NMR spectra data (60 MHz, $CDCl_3$, δ value) |
|---|---|
| 16 | 1.1 (6H, s), 1.1 (6H, t), 2.1 (3H, s), 2.5 (4H, s), 2.6 (4H, m), 5.2 (1H, s) 6.3 (1H, s) |
| 19 | 1.1 (6H, s), 1.1 (3H, t), 2.1 (3H, s) 2.5 (4H, s), 2.5 (8H, m), 5.1 (1H, s), 6.4 (1H, s) |
| 20 | 1.1 (6H, s), 1.2 (3H, t), 1.8 (5H, m), 2.1 (3H, s), 2.5 (4H, s), 2.8 (4H, m), 4.1 (1H, q), 5.1 (1H, s), 6.3 (1H, s) |
| 25 | 1.1 (9H, s), 2.1 (3H, s), 2.3 (6H, s), 2.6 (5H, m), 5.2 (1H, s), 6.3 (1H, s) |

Next, the present invention is described with reference to example below but is not deemed to be limited thereto.

Example 1

2-((4,5-bis(dimethylamino)-1,3-dithiolan)-2-ylidene)-1,3-dioxocyclohexane (compound No. 1)

1,3-cyclohexanedione, 2.24 g (0.02 mole) and 1.6 g (0.02 mole) of carbon disulfide were dissolved in 5 mℓ of dimethylformamide. Under ice cooling, 1.8 g of potassium hydroxide powders were added to the solution. The mixture was stirred for an hour at the same temperature to prepare a dithiolate solution. On the other hand, 5.68 g (0.0 mole) of glyoxal sodium bisulfite adduct was dissolved in a 30 mℓ of water in a separate flask and was cooled in a ice-salt bath and 4.32 g (0.048 mole) of 50% dimethylamine aqueous solution was dropwise added to the solution until the mixture became a homogeneous solution. The dithiolate solution previously prepared was dropwise added to the solution while cooling. The mixture was stirred for additional minutes at the same temperature. The precipitated crystals were collected by filtration and air-dried. Crude crystals were recrystallized from chloroform-ether to give 1.92 g of the desired product. yield 29%; m.p. 175 ~ 178° C

Example 2

2-((4-hydroxy-5-dimethylamino-1,3-dithiolan)-2-ylidene)-1,3-cyclohexanedione (compound No. 5)

1,3-cyclohexanedione, 28.5 g (0.25 mole) was dissolved in 60 mℓ of dimethylsulfoxide. 36.2 g (0.55 mole) of potassium hydroxide powder was added to the solution and the mixture was stirred for 5 minutes. While keeping at 30 ~ 40° C, 20.9 g (0.275 mole) of carbon disulfide was dropwise added and the mixture was stirred for 30 minutes at the same temperature to prepare a dithiolate solution. On the other hand, 36.3 g (0.25 mole) of 40% glyoxal aqueous solution, 45 g (0.5 mole) of 50% dimethylamine aqueous solution and 480 mℓ of water was placed in a flask and 46 mℓ (0.5 mole) of conc. hydrochloric acid was dropwise added to the mixture. The mixture was stirred for 30 minutes at the same temperature. The dithiolate solution previously prepared was dropwise added to the aqueous solution under ice cooling. The mixture was stirred for 30 minutes at the same temperature. The precipitated crystals were collected by filtration and crude crystals was dissolved in 1 ℓ of chloroform. After drying over magnesium sulfate, the solvent was distilled off to give 32 g of crude crystals. The crude crystals were washed with 0.1 N acetic acid, followed by recrystallized from methylene chloride to give 18.4 g of the desired product.
yield 27%; m.p. 153 ~ 156° C

Example 3

2-((4-acetoxy-5-dimethylamino-1,3-dithiolan)-2-ylidene)-1,3-cyclohexanedione (compound No. 6)

2-((4-hydroxy 5-dimethylamino-1,3-dithiolan)- 2-ylidene)-1,3-cyclohexanedione, 13.7 g (0.05 mole) was dissolved in 100 mℓ of chloroform. 20 mℓ of pyridine was added to the solution, and 11.0 g (0.14 mole) of acetyl chloride was then dropwise added to the mixture under ice-cooling and the mixture was stirred for 30 minutes. The reaction solution was washed with 100 mℓ of 0.1 N hydrochloric acid and 100 mℓ of water. Organic phase was dried over magnesium sulfate and distilled off to give crude crystals. Crude crystals were recrystallized from ethyl acetate-n-hexane to give 0.5 g of pale yellow crystals.
yield 60%; m.p. 148 ~ 149 °C

Example 4

2-((4-acetoxy-5-(4-methylpiperazino)-1,3-dithiolan)-2-ylidene)-5-(4-methylphenyl)-1,3-cyclohexanedione (compound No. 32)

20.2 g (0.10 mole) of 5-(4-methylphenyl)-1,3-cyclohexanedione 8.4 g (0.11 mole) of carbon disulfide was dissolved in 60 mℓ of dimethylsulfoxide. 29.0 g (0.22 mole) of 50% potassium hydroxide aqueous solution was dropwise added to the solution at 30 ~ 35 °C under cooling, and the mixture was stirred for 1 hour to prepare a dithiolate solution. On the other hand, 200 g of ice was added to a solution of 14.5 g (0.10 mole) of 40% glyoxal aqueous solution in a separate flask, and 22.0 g (0.22 mole) of N-methylpiperazine and 21.6 g of hydrochloric acid was added to the solution and the mixture was stirred for 40 minutes under ice-cooling. The dithiolate solution previously prepared was dropwise added to the solution and the mixture was stirred for 30 minutes at the same temperature. The precipitated crystals were collected by filtration and crude crystals were dissolved in 1 ℓ of methylene dichloride, washed with water and dried over magnesium sulfate. The solvent was distilled off to give 17.0 g of crude crystals of 2-((4-hydroxy-5-(4-methylpiperazino)-1,3-dithiolan)-2-ylidene)-5-(4-methylphenyl)-1,3-cyclohexanedione. To the suspension of the crystals in 200 mℓ methylene dichloride was added 12.8 g (0 13 mole) of triethylamine in one portion and dropwise 5.0 g (0.06 mole) of acetyl chloride under ice cooling. The mixture was stirred for 30 minutes. Reaction solution was washed with water and organic layer was dried over magnesium sulfate. The solvent was distilled off and the residue was crystallized from ethyl acetate. The obtained crystals were purified by silica gel chromatography and then recrystallized from ethyl acetate to give 11.0 g of the desired product.
yield 26.0%, m.p. 189 ~ 190 °C

Example 5

2-((4-acetoxy- 5-dimethylamino-1,3-dithiolan)-2-ylidene)-5-(4 chlorophenyl)-1,3-cyclohexanedione (compound No. 35)

To a solution of 5-(2-chlorophenyl)-1,3- cyclohexanedione 11.1 g (0.05 mole) and 4.2 g (0.055 mole) of carbon disulfide in 38 mℓ of dimethysulfoxide was added solution of 7.2 g of potassium hydroxide powder under cooling and the mixture was stirred for 1 hour at room temperature. On the other hand, 10 g of 50% dimethylamine aqueous solution was dropwise added to the ice cooled aqueous solution of 7.3 g (0.05 mole) of 40% glyoxal aqueous solution in a 100 mℓ of water under ice-cooling. Subsequently, 10.8 g (0.11 mole) of conc. hydrochloric acid was dropwise added to the solution. After the mixture was stirred for 40 minutes at the same temperature, the dithiolate solution previously prepared was dropwise added to the solution under ice-cooling and the mixture was stirred for 40 minutes at the same temperature. The precipitated crystals were collected by filtration and dried to give 14.0 g of crude crystals. To the solution of the crude crystals in 300 mℓ of chloroform and 40 mℓ of pyridine was dropwise added 10.0 g (0.13 mole) of acetyl chloride under ice cooling. After the mixture was stirred for 20 minutes, it was extracted with chloroform. The extract was washed with 1 N hydrochloric acid, diluted sodium hydrogen carbonate aqueous solution and water. After drying the organic phase over magnesium sulfate, the solvent was

distilled off. The obtained products were purified by silicagel chromatography (ethyl acetate-n-hexane), and then recrystallized from ethyl acetate-n-hexane to give 6.4 g of the desired product.
yield 30 %, m.p. 154 ~ 157° C

Example 6

2-((4-hydroxy-5-dimethylamino-1,3-dithiolan)-2-ylidene)-5-(2-furyl)-1,3 cyclohexanedione (compound No. 51)

Under ice-cooling, 6.5 g of potassium hydroxide powder was added to a mixture of 8.9 g (0.05 mole) of 5-(2-furyl) 1,3-cyclohexanedione and 4.2 g (0.055 mole) of carbon disulfide in 50 mℓ of dimethysulfoxide and the mixture was stirred for 30 minutes at the same temperature. On the other hand, 10 g of 50 % dimethylamine aqueous solution was dropwise added to a ice cooled aqueous solution of 7.3 g (0.05 mole) of 40 % glyoxal aqueous solution in a 100 mℓ of water, the reaction temperature being maintained at 0 ~ 5° C. Subsequently, 9 g (0.11 mole) of conc. hydrochloric acid was dropwise added to the mixture at 0 ~ 5° C. After reaction mixture was stirred for 30 minutes at the same temperature, the dithiolate solution previously prepared was dropwise added to the solution at 0 ~ 5° C and the mixture was stirred for 15 minutes at the same temperature. Reaction solution was extracted with ethyl acetate, washed with water three times. The extract was dried over magnesium sulfate. The solvent was distilled off to give 6.5 g of crude crystals.
Yield 38 %, m.p. 121 ~ 132° C

Example 7

2-((4-acetoxy-5-dimethylamino-1,3-dithiolan)-2-ylidene)-5-(2-furyl)-1,3-cyclohexanedione (compound No. 45)

Under ice-cooling, 30 mℓ of triethylamine was added to a solution of 13 g (0.038 mole) of 2-(4-hydroxy-5-dimethylamino-1,3-dithiolan-2-ylidene)-5-(2-furyl)-1,3-cyclohexanedione in 500 mℓ of methylene dichloride. Subsequently 12 g (0.15 mole) of acetyl chloride was dropwise added to the mixture at 5 ~ 10° C. After the dropwise addition, the mixture was stirred for minutes at the same temperature. Water was added to the reaction solution and organic phase was separated and washed with water. After drying organic phase over magnesium sulfate, the solvent was distilled off to give crude products. It was purified by silica gel chromatography (ethyl acetate n-hexane) and then recrystallized from ether to give 12 g of pale yellow crystals.
yield 78 %, m.p. 159 ~ 160° C

The dithiolane derivatives represented by general formula (I) have such a low toxicity that even when these compounds are administered to mice in a dose of 300 mg/kg/day for consecutive several days, the mice neither show toxic symptoms nor die.

The compounds represented by general formula (I) are useful as drugs for curing hepatic diseases. For example it is known that hepatic disorders can be caused in an experimental animal by giving various chemicals such as carbon tetrachloride, etc. (for example, Japanese Published Patent Application KOKOKU No. 18579/1981). It has been found that the compounds represented by general formula (I) and salts thereof show marked inhibitory or improving effects against reduced liver functions in the animal suffering from experimental hepatic disturbances of various morbid models when administered orally or parenterally (e.g., injection). Therefore, the compounds represented by general formula (I) are useful as drugs applied to human and animals for treatment or prophylaxis of hepatic diseases. That is, the compounds can be used as agents for treating acute or chronic liver diseases which occur on human or animal due to various causes, for example, fatty liver, alcohol-induced hepatitis, hepatitis, intoxicative liver diseases, congestion of liver, bile congestive hepatic disturbance or liver cirrhosis which is the terminal pattern of these diseases.

Accordingly, the term "drug for treatment of hepatic diseases" as used in the present specification refers to a drug for treatment or/and prophylaxis of hepatic diseases utilizing pharmacological activities such as the aforesaid liver function activating activity exerted on liver and prophylactic and therapeutic activities of hepatic diseases, etc.

The compounds represented by general formula (I) can be used as they are, as drugs for curing

hepatic diseases. It is also possible to formulate them into mixtures with pharmaceutically acceptable diluents and/or other pharmacologically active ingredients according to pharmaceutical custom. Furthermore, they can also be formulated into dosage unit forms. Forms which they can have as drugs include powders, granules, tablets, dragees, capsules, pills, suspensions, solutions, emulsions, ampoules, injections, isotonic solutions, etc.

Formulation of the compound of this invention into a medicinal composition includes an embodiment in which the compound represented by general formula (I) is incorporated into the composition in the form of a mixture with pharmaceutically acceptable diluents. The term "diluents" used herein means materials other than the compound represented by general formula (I). The diluents may be any of solids, semisolids, liquids and ingestible capsules and include various materials, for example, excipients, extenders, binders, wetting agents, disintegrators, surfactants, lubricants, dispersants, buffers, taste-improver, odour-reducing agents, coloring matters, flavors, preservatives, dissolution assistance, solvents, coatings, frostings, etc. But the diluents are not limited thereto. These materials are used alone or as a mixture thereof. Such pharmaceutically acceptable diluents are used as a mixture with other pharmacologically active ingredients in some cases.

A medicinal composition using the compound of this invention may be produced by any known method. For example, the active ingredient is mixed with diluents to yield, for instance, granules, and then the composition thus obtained is formed, for example, into tablets. When the medicinal composition is used as a parenteral drug, it should be sterilized. If necessary, it should be made isotonic with regard to blood.

In this invention, since the compounds represented by above general formula (I) themselves are applicable as drugs for curing hepatic diseases, the active ingredient is contained in the composition usually in an amount of 0.01 to 100% (by weight).

When the compound of this invention is formulated into a preparation of dosage unit, individual pharmaceutical portions constituting said preparation may be either in different forms or in the same forms, and there are often employed, for example, forms such as tablets, granules, pills, powders, dragees, capsules, and ampoules.

The drugs for curing hepatic diseases according to this invention can be applied to human beings and animals in order to prevent and cure hepatic disorders, by a method which is conventional in the fields of such prevention and therapy. They are administered orally or parenterally. The oral administration includes sublingual administration. The parenteral administration includes administration by injection (including, for example, subcutaneous injection, intramuscular injection, intravenous injection, and drip).

The dose of the drugs of this invention is varied depending various factors such as animals or human beings of subject, sensitivity difference, age, sex and body weight, the administration route, time and interval of administration, condition of a disease, physical condition of the subject, properties of pharmaceutical preparation, kind of preparation, kind of active ingredient, etc.

Therefore, in some cases, a dose smaller than the minimum dose described below is sufficient, and in other cases, a dose larger than the maximum dose described below is required.

In the case of a high dose, administration in several times a day is preferred.

In order to obtain effective results for animals, the dose in terms of the active ingredient is advantageously 0.1 to 300 mg, preferably 0.1 to 30 mg per kg of body weight per day in the case of oral administration, while in the case of parenteral ministration, it is advantageously 0.01 to 250 mg, preferably 0.1 to 25 mg per kg of body weight per day.

In order to obtain effective results for human beings, in consideration of sensitivity difference, safety, etc. on the basis of the effective dose for animals, the dose for human beings seems to be advantageously, for example, in the following ranges: in the case of oral administration, 0.1 to 200 mg, preferably 0.5 to 50 mg per kg of body weight per day, and in the case of parenteral administration, 0.01 to 100 mg, preferably 0.1 to 25 mg per kg of body weight per day.

Hereafter, several examples of medical formulations are shown below but the present invention is not deemed to be limited thereto.

In the following formulation examples, all parts are by weight.

| Formulation Example 1 | |
| --- | --- |
| Compound 2 | 10 parts |
| Heavy magnesium oxide | 10 parts |
| Lactose | 80 parts |

A powder or fine granular preparation was prepared by mixing uniformly and pulverizing or granulating finely the above ingredients.

| Formulation Example 2 | |
|---|---|
| Compound 7 | 10 parts |
| Synthetic aluminum silicate | 10 parts |
| Calcium hydrogenphosphate | 5 parts |
| Lactose | 75 parts |

A powder was prepared according to Formulation Example 1 by using the above ingredient.

| Formulation Example 5 | |
|---|---|
| Compound 13 | 50 parts |
| Starch | 10 parts |
| Lactose | 15 parts |
| Crystalline cellulose | 20 parts |
| Polyvinyl alcohol | 5 parts |
| Water | 30 parts |

Granules were prepared by kneading together uniformly, grinding, granulating the above ingredients, drying the resultant, and then sieving.

Formulation Example 4

Tablets having a diameter of 10 mm were prepared by mixing 99 parts of the granules obtained in Formulation Example 3 with 1 part of calcium stearate, and compression-molding the resulting mixture.

| Formulation Example 5 | |
|---|---|
| Compound 30 | 78 parts |
| Polyvinyl alcohol | 2 parts |
| Lactose | 20 parts |
| Water | 30 parts |

Granules were prepared in the same manner as in Formulation Example 3 except for using the above ingredients. To 90 parts of the granules obtained was added 10 part of crystalline cellulose, and the resulting mixture was compression-molded into tablets having a diameter of 8 mm. Then, the tablets were made into dragees by use of suitable amounts of a mixed suspension of syrup, gelatin and precipitated calcium carbonate and coloring matter.

| Formulation Example 6 | |
|---|---|
| Compound 46 | 0.5 parts |
| Nonionic surface active agent | 2.5 parts |
| Physiological saline | 97 parts |

An injection was prepared by mixing by heating, and then sterilizing the above ingredients.

Formulation Example 7

Capsules were prepared by packing the powder obtained in Formulation Example 1 into commercially available capsular containers.

Next, in order to prove the effectiveness of the compounds of this invention, the test example is shown below.

Test Example 1

Effect of inhibiting carbon tetrachloride-induced hepatic disorders

Method:

A test compound was dissolved or suspended in olive oil and the resulting solution or suspension was orally administered to male mice (6 week age) of dd strain in a dose of 30 mg/kg. Six hours after the administration, carbon tetrachloride was orally administered to the mice in a dose of 0.05 ml/kg. The mice were sacrificed 24 hours after the administration of carbon tetrachloride. Degree of hepatic disorders was visually examined.

On the other hand, blood was collected when they were sacrificed and plasma was obtained by centrifugation. Plasma glutamic-pyruvic transaminase (GPT) activity was determined according to the Reitman-Frankel method and the activity was expressed by Karmen Unit (K.U.). The hepatic disorder index is evaluated as below.

| Hepatic Disorder Index | Condition of Liver |
|---|---|
| 0 | Healthy liver |
| 2 | Slightly influenced |
| 4 | Disorder was obviously noted. |
| 6 | Heavy disorder |

Five (5) mice were used in each group and the results are shown by a mean value. Further with respect to GPT activity, no further measurement was conducted with the case showing more than 2,100 units but for brevity, it was calculated as 2,100 units.

The results are shown in Table 3.

Table 3

| Activity on Carbon Tetrachloride-Induced Hepatic Disorder | | |
|---|---|---|
| Compound No of this Invention | Hepatic Disorder index | p-GPT(K.U.) |
| Single Administration of Carbon Tetrachloride | 6.0 | 2,100 |
| Intact | 0 | 14 |
| Compound No. 1 | 0.6 | 17 |
| Compound No. 2 | 0.5 | 12 |
| Compound No. 3 | 1.6 | 49 |
| Compound No. 7 | 0 | 15 |
| Compound No. 8 | 0 | 18 |
| Compound No. 13 | 0.4 | 157 |
| Compound No. 19 | 1.0 | 297 |
| Compound No. 30 | 0.5 | 229 |
| Compound No. 46 | 0.2 | 205 |
| Compound No. 47 | 1.6 | 430 |

As is noted from the results above, the compounds of this invention show activating action on liver functions and are thus effective as liver function activaters or therapeutic agents for hepatic disease in human or animal.

While the invention has been described in detail and with reference to specific embodiments thereof, it will be apparent to one skilled in the art that various changes and modifications can be made therein without departing from the spirit and scope thereof.

## Claims

1. A dithiolane derivative represented by the general formula (I)

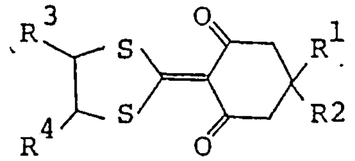

(I)

wherein $R^1$ represents hydrogen atom; $C_1$-$C_6$ alkyl group; phenyl group; phenyl group substituted with 1 to 2 groups selected from the group consisting of halogen atom, $C_1$-$C_4$ alkyl group, $C_1$-$C_4$ alkoxy group, $C_1$-$C_4$ haloalkoxy group, $C_1$-$C_4$ alkylthio group and methylenedioxy group; furyl group which may or not may be substituted with $C_1$-$C_4$ alkyl group, or thienyl group; $R^2$ represent hydrogen atom or $C_1$-$C_4$ alkyl group, $R^3$ represents di($C_1$-$C_4$ alkyl)amino group, N-hydroxyethyl N-methylamino group, formula:

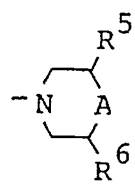

(wherein $R^5$ and $R^6$, which may be the same or different, represent hydrogen atom or $C_1$-$C_4$ alkyl group, A represent nitrogen atom substituted with $C_1$-$C_4$ alkyl group or $C_2$-$C_5$ alkoxycarbonyl group, oxygen atom, methylene group or single bond, $R^4$ has a same meaning as $R^3$, hydroxy group or formula:

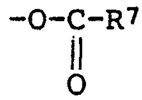

(wherein $R^7$ represents $C_1$-$C_4$ alkyl group, $C_1$-$C_4$ alkoxy group, furyl group, phenyl group, p-chlorophenyl group) or its pharmaceutically acceptable salts.

2. A dithiolane derivative or pharmaceutically acceptable salt thereof according to claim 1, wherein $R^1$ represents hydrogen atom; $C_1$-$C_6$ alkyl group; phenyl group; phenyl group substituted with 1 to 2 groups selected from the group consisting of halogen atom, $C_1$-$C_4$ alkyl group and $C_1$-$C_4$ alkoxy group; or furyl group which may or not may be substituted with $C_1$-$C_4$ alkyl group; $R^2$ represents hydrogen atom or $C_1$-$C_4$ alkyl group, R3 represents di($C_1$-$C_4$ alkyl) amino group, morpholino group or 4-$C_1$-$C_4$ alkylpiperazino group, $R^4$ represents hydroxy group, $C_2$-$C_5$ alkylcarbonyloxy group or $C_2$-$C_5$ alkoxycarbonyloxy group.

3. A dithiolane derivative or pharmaceutically acceptable salt thereof according to claim 1 or 2, wherein $R^1$ represents hydrogen atom; methyl group; phenyl group; tolyl group; furyl group; 2-methyl furyl group; $R^2$ represents hydrogen atom or methyl group, $R^3$ represents dimethylamino group, morpholino group or 4-methylpiperazino group, $R^4$ represents acetoxy group, propionyloxy group or ethoxycarbonyloxy group.

4. A dithiolane derivative or pharmaceutically acceptable salt thereof according to claim 1, wherein $R^1$ represents hydrogen atom; $C_1$-$C_6$ alkyl group; phenyl group or phenyl group substituted with 1 to 2 groups selected from the group consisting of halogen atom, $C_1$-$C_4$ alkyl group and $C_1$-$C_4$ alkoxy group; $R^2$ represents hydrogen atom or $C_1$-$C_4$ alkyl group, $R^3$ and $R^4$, which may be the same or different, represent di($C_1$-$C_4$ alkyl) amino group, morpholino group or 4-$C_1$-$C_4$ alkylpiperazino group.

5. A dithiolane derivative or pharmaceutically acceptable salt thereof according to claim 1 or 4, wherein $R^1$ and $R^2$, which may be the same or different, represents hydrogen atom or $C_1$-$C_6$ alkyl group; $R^3$ and $R^4$, which may be the same or different, represent di($C_1$-$C_4$ alkyl) amino group or morpholino group.

6. A dithiolane derivative or pharmaceutically acceptable salt thereof according to claim 1, 4, or 5 , wherein $R^1$ and $R^2$ represent hydrogen atom, $R^3$ and $R^4$, which may be the same or different, represent dimethylamino group or morpholino group.

7. A dithiolane derivative or pharmaceutically acceptable salt thereof according to claim 1, 4 or 5 wherein $R^1$ represents methyl group; $R^2$ represents hydrogen atom or methyl group, $R^3$ and $R^4$, which may be the same or different, represents dimethylamino group or morpholino group.

8. A dithiolane derivative as in any one of claims 1, 2 or 3 which is 2-((4-acethoxy-5-dimethylamino-1,3-dithiolan)-2-ylidene)-1,3-cyclohexanedione.

9. A dithiolane derivative as in any one of claims 1,2 or 3 which is 2-((4-propionyloxy-5-dimethylamino-1,3-dithiolan)-2-ylidene)-1,3-cyclohexanedione.

10. A dithiolane derivative as in any one of claims 1,2 or 3 which is 2-((4-acethoxy-5-(4-methylpiperazino) 1,3-dithiolan)-2-ylidene)-1,3-cyclohexanedione.

11. A dithiolane derivative as in any one of claims 1,4,5 or 6 which is 2-((4,5-bis(dimethylamino)-1,3-dithiolan)2-ylidene)-1,3-dioxocyclohexane.

12. A dithiolane derivative as in any one of claims 1,4,5 or 6 which is 2-((4,5-dimorpholino-1,3-dithiolan)-2-ylidene)-1,3-dioxocyclohexane.

13. A composition for treating hepatic disease comprising as an effective ingredient a dithiolane derivative represented by the general formula (I)

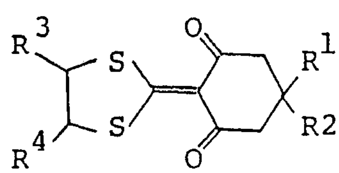

(I)

wherein $R^1$ represents hydrogen atom; $C_1$-$C_6$ alkyl group; phenyl group; phenyl group substituted with 1 to 2 groups selected from the group consisting of halogen atom, $C_1$-$C_4$ alkyl group, $C_1$-$C_4$ alkoxy group, $C_1$-$C_4$ haloalkoxy group, $C_1$-$C_4$ alkylthio group and methylenedioxy group; furyl group which may or not may be substituted with $C_1$-$C_4$ alkyl group, or thienyl group; $R^2$ represents hydrogen atom or $C_1$-$C_4$ alkyl group, $R^3$ represents di($C_1$-$C_4$ alkyl)amino group, formula:

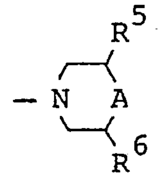

(wherein $R^5$ and $R^6$, which may be the same or different represent hydrogen atom or $C_1$-$C_4$ alkyl group, A represents nitrogen atom substituted with $C_1$-$C_4$ alkyl group or $C_2$-$C_5$ alkoxycarbonyl group, oxygen atom, methylene group or single bond, $R^4$ have a same meaning as $R^3$, hydroxy group or formula

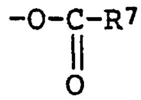

(wherein $R^7$ represents $C_1$-$C_4$ alkyl group, $C_1$-$C_4$ alkoxy group, furyl group, phenyl group, p-chlorophenyl group) or its pharmaceutically acceptable salts.

14. A composition for treating hepatic disease according to claim 13, wherein $R^1$ represents hydrogen atom; $C_1$-$C_6$ alkyl group; phenyl group; phenyl group substituted with 1 to 2 groups selected from the group consisting of halogen atom, $C_1$-$C_4$ alkyl group and $C_1$-$C_4$ alkoxy group; or furyl group which may or not may be substituted with $C_1$-$C_4$ alkyl group; $R^2$ represents hydrogen atom or $C_1$-$C_4$ alkyl group, $R^3$ represents di($C_1$-$C_4$ alkyl)amino group, morphilino group or 4-$C_1$-$C_4$ alkylpiperazino group, $R^4$ represents hydroxy group, $C_2$-$C_5$ alkylcarbonyloxy group or $C_2$-$C_5$ alkoxycarbonylox group.

15. A composition for treating hepatic disease according to claim 13 or 14 wherein $R^1$ represents hydrogen atom; methyl group; phenyl group; tolyl group; furyl group; 2-methyl furyl group; $R^2$ represents hydrogen atom or methyl group, $R^3$ represents dimethylamino group, morpholino group or 4-methylpiperazino group, $R^4$ represents acetoxy group, propionyloxy group or ethoxycarbonyloxy group.

16. A composition for treating hepatic disease according to claim 13, wherein $R^1$ represents hydrogen atom; $C_1$-$C_6$ alkyl group; phenyl group or phenyl group substituted with 1 to 2 groups selected from the group consisting of halogen atom, $C_1$-$C_4$ alkyl group and $C_1$-$C_4$ alkoxy group; $R^2$ represents hydrogen atom or $C_1$-$C_4$ alkyl group, $R^3$ and $R^4$, which may be the same or different, represent di($C_1$-$C_4$ alkyl) amino group, morpholino group or 4-$C_1$-$C_4$ alkylpiperazino group.

17. A composition for treating hepatic disease according to claim 13 or 16, wherein $R^1$ and $R^2$, which may be the same or different, represent hydrogen atom or $C_1$-$C_6$ alkyl group; $R^3$ and $R^4$, which may be the same or different, represent di($C_1$-$C_4$ alkyl) amino group or morpholino group.

18. A composition for treating hepatic disease according to claim 13, 16 or 17, wherein $R^1$ and $R^2$ represent hydrogen atom, $R^3$ and $R^4$, which may be the same or different, represent dimethylamino group or morpholino group.

19. A composition for treating hepatic disease according to claim 13, 16 or 17, wherein $R^1$ represents methyl group; $R^2$ represents hydrogen atom or methyl group, $R^3$ and $R^4$, which may be the same or different, represent dimethylamino group or morpholino group.

20. A pharmaceutical composition as in any one of claims 13, 14 or 15 which is said dithiolane derivative is 2-((4-acethoxy-5-dimethylamino-1,3-dithiolan)-2-ylidene)-1,3-cyclohexanedione.

21. A pharmaceutical composition as in any one of claims 13, 14 or 15 which is said dithiolane derivative is 2-((4-propionyloxy-5-dimethylamino-1,3-dithiolan)-2-ylidene)-1,3-cyclohexanedione.

22. A pharmaceutical composition as in any one of claims 13, 14 or 15 which is said dithiolane derivative is 2-((4-acethoxy-5-(4-methylpiperazino)-1,3-dithiolan)-2-ylidene) 1,3 cyclohexanedione.

23. A pharmaceutical composition as in any one of claims 13, 16 or 17 which is said dithiolane derivative is 2-((4,5-bis(dimethylamino)-1,3-dithiolan)-ylidene)-1,3-dioxocyclohexane.

24. A pharmaceutical composition as in any one of claims 13, 16 or 17 which is said dithiolane derivative is 2-((4,5-dimorpholino-1,3-dithiolan)-2-ylidene)-1,3-dioxocyclohexane.

25. A process for producing a dithiolane derivative represented by the general formula (Ia)

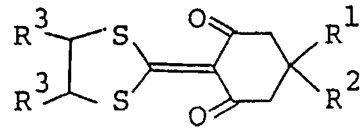

(Ia)

(Ia)

wherein:

$R^1$ represents hydrogen atom; $C_1$-$C_6$ alkyl group; phenyl group; phenyl group substituted with 1 to 2 groups selected from the group consisting of halogen atom, $C_1$-$C_4$ alkyl group, $C_1$-$C_4$ alkoxy group, $C_1$-$C_4$ haloalkoxy group, $C_1$-$C_4$ alkylthio group and methylenedioxy group; furyl group which may or not may be substituted with $C_1$-$C_4$ alkyl group, or thienyl group; $R^2$ represents hydrogen atom or $C_1$-$C_4$ alkyl group, $R^3$ represents di($C_1$-$C_4$ alkyl)amino group, N-hydroxyethyl N-hydroxyethyl N-methylamino group, formula:

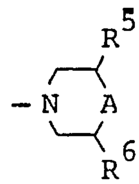

(wherein $R^5$, which may be the same or different, hydrofgen atom or $C_1$-$C_4$ alkyl group, A represents nitrogen atom substituted with $C_1$-$C_4$ alkyl group or $C_2$-$C_5$ alkoxycarbonyl group, oxygen atom, methylene group or single bond, which comprises reacting glyoxal sodium bisulfite adduct represented by structural formula:

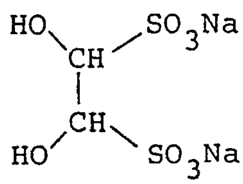

with a compound represented by the general formula (II):

$R^3$ - H (II)

wherein $R^3$ has the same significance as described above, and then reacting with a compound represented by the general formula (III):

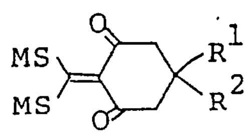

(III)

wherein $R^1$ and $R^2$ have the same significance as described above and M represents an alkali metal atom.

26. A process for producing a dithiolane derivative represented by the general formula (Ib)

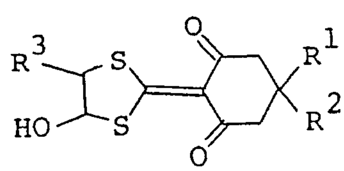

(Ib)

wherein:

$R^1$ represents hydrogen atom; $C_1$-$C_6$ alkyl group; phenyl group; phenyl group substituted with 1 to 2 groups selected from the group consisting of halogen atom, $C_1$-$C_4$ alkyl group, $C_1$-$C_4$ alkoxy group, $C_1$-$C_4$

haloalkoxy group, $C_1$-$C_4$ alkylthio group and methylenedioxy group; furyl group which may or not may be substituted with $C_1$-$C_4$ alkyl group, or thienyl group; $R^1$ represents hydrogen atom or $C_1$-$C_4$ alkyl group, $R^3$ represents di($C_1$-$C_4$ alkyl) amino group, N-hydroxyethyl N-methylamino group, formula:

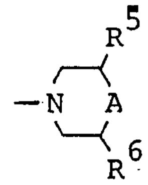

(wherein $R^5$, which may be the same or different, hydrogen atom or $C_1$-$C_4$ alkyl group, A represents nitrogen atom substituted with $C_1$-$C_4$ alkyl group or $C_2$-$C_5$ alkoxycarbonyl group, oxygen atom, methylene group or single bond, which comprises reacting glyoxal with a compound represented by the general formula (II):

$R^3$-H (II)

wherein $R^3$ has the same significance as described above, in the presence of the acid, and then reacting with a compound represented by the general formula (III)

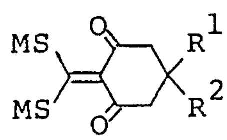

(III)

wherein $R^1$ and $R^2$ have the same significance as described above and M represents an alkali metal atom.

27. A process for producing a dithiolane derivative represented by the general formula (Ic)

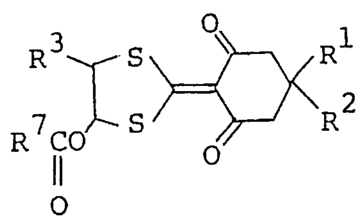

(Ic)

wherein:

$R^1$ represents hydrogen atom; $C_1$-$C_6$ alkyl group; phenyl group; phenyl group substituted with 1 to 2 groups selected from the group consisting of halogen atom, $C_1$-$C_4$ alkyl group, $C_1$-$C_4$ alkoxy group, $C_1$-$C_4$ haloalkoxy group, $C_1$-$C_4$ alkylthio group and methylenedioxy group; furyl group which may or not may be substituted with $C_1$-$C_4$ alkyl group, or thienyl group; $R^1$ represents hydrogen atom or $C_1$-$C_4$ alkyl group, $R^3$ represents di($C_1$-$C_4$ alkyl) amino group, N-hydroxyethyl N-methylamino group, formula:

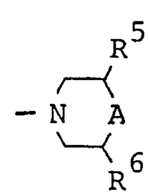

(wherein $R^6$, which may be the same or different, represent hydrogen atom or $C_1$-$C_4$ alkyl group, A represents nitrogen atom substituted with $C_1$-$C_4$ alkyl group or $C_2$-$C_5$ alkoxycarbonyl group, oxygen atom, methylene group or single bond, $R^7$ represents $C_1$-$C_4$ alkyl group, $C_1$-$C_4$ alkoxy group, furyl group, phenyl group, p-chlorophenyl group, which comprises reacting a compound represented by the general formula (Ib) with a compound represented by the general formula (V):

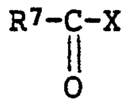

(V)

wherein $R^7$ has the same significance as described above, X represents a halogen atom, in the presence of the base.

European Patent Office

# EUROPEAN SEARCH REPORT

Application Number

EP 89 11 6152

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 261 674 (NIHON NOHYAKU CO.)<br>* Page 3, lines 4-25,47-48; page 4, lines 7-41; pages 8-24, table 1; page 25, lines 13-24; pages 26-29, examples 1-6,8; claims 1,14,16 * & JP-A-63 183 586 | 1,4-7, 13,16-19,25 | C 07 D 339/06<br>C 07 D 409/04<br>C 07 D 409/08<br>C 07 D 409/12<br>A 61 K 31/385 |
| A | CHEMICAL ABSTRACTS, vol. 109, no. 19, November 1988, page 722, column 1, abstract no. 170411w, Columbus, Ohio, US; Y. TSUCHIYA et al.: "Preparation of 2-substituted 1,3-dithiolane derivatives and their use for treating liver diseases and disorders", & JP-A-63 027 488 (BANYU PHARMACEUTICAL CO.) | 1-3,13-15,25, 26 | |
| | | | TECHNICAL FIELDS SEARCHED (Int. Cl.5) |
| | | | C 07 D 339/00<br>C 07 D 409/00 |

The present search report has been drawn up for all claims

| Place of search | Date of completion of the search | Examiner |
|---|---|---|
| THE HAGUE | 20-03-1990 | ENGLISH R.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document